# EUROPÄISCHE PATENTSCHRIFT

(11) **EP 4 304 503 B1**
(45) Veröffentlichungstag und Bekanntmachung des Hinweises auf die Patenterteilung: **14.05.2025**
(21) Anmeldenummer: 22710560.8
(22) Anmeldetag: 03.03.2022
(51) Int. Cl.: A61B 17/80

(54) **OSTEOSYNTHESEPLATTE ALS ERSATZ EINER SYNARTHROSE IN FORM EINER SYMPHYSE**
OSTEOSYNTHESIS PLATE AS REPLACEMENT OF A SYNARTHROSIS IN THE FORM OF A SYMPHYSIS
PLAQUE ORTHOPÉDIQUE POUR REMPLACER UNE SYNARTHROSE EN FORME D'UNE SYMPHYSE

(30) Priorität: 11.03.2021 EP 21162117
(43) Veröffentlichungstag der Anmeldung: 17.01.2024
(73) Patentinhaber: Universitätsspital Basel, 4031 Basel (CH)
(72) Erfinder: ECKARDT, Henrik, 4102 Binningen (CH)
(74) Vertreter: Kleine, Hubertus
(86) Internationale Anmeldenummer: PCT/EP2022/055474
(87) Internationale Veröffentlichungsnummer: WO 2022/189267

(56) Entgegenhaltungen:
- EP-A1- 2 494 934
- CN-A- 112 107 356
- DE-A1- 102012 010 024
- DE-B1- 2 603 087
- DE-B3- 102005 032 026
- US-A1- 2005 165 401

## Beschreibung

Die vorliegende Erfindung betrifft eine Osteosyntheseplatte nach dem Oberbegriff des Anspruchs 1.

Osteosyntheseplatten, welche sich als Ersatz einer Synarthrose eignen, sind typischerweise streifenförmige oder als L-abgewinkelte flache Metallleisten. Problematisch ist, dass ein Becken oder andere Körperteile einer ständigen Bewegung unterliegen. Bei der Bewegung wirken in der Ebene der Metallleiste bei Bewegung entsprechende Zug- und Druckkräfte, die sich auf die Schrauben übertragen werden. Dadurch wird die Knochenschraube beständig hin und her bewegt, so dass sie das Bohrloch innerhalb des Knochens erweitert und sich die Schraube dadurch aus der Verankerung des Knochens lösen kann oder es kann zu einem Schraubenbruch oder Plattenbruch kommen.

Die DE 26 03 087 B1 offenbart eine gattungsgemäße Osteosyntheseplatte mit Fixationsplattensegmenten zur Stabilisierung einer Symphyse, welche jeweils eine Auflagenfläche zur Auflage an einem Beckenrand aufweisen und ein daraus hervorstehendes Verbindungssegment, welches derart aus dem Fixationsplattensegmenten hervorsteht, dass es geeignet ist zur Erstreckung entlang der Beckeninnenseite eines Menschen. Das Material der Osteosymphyseplatte ist thermoplastisch und wird durch Hitze weich und passt sich die Anatomie der Symphyse an.

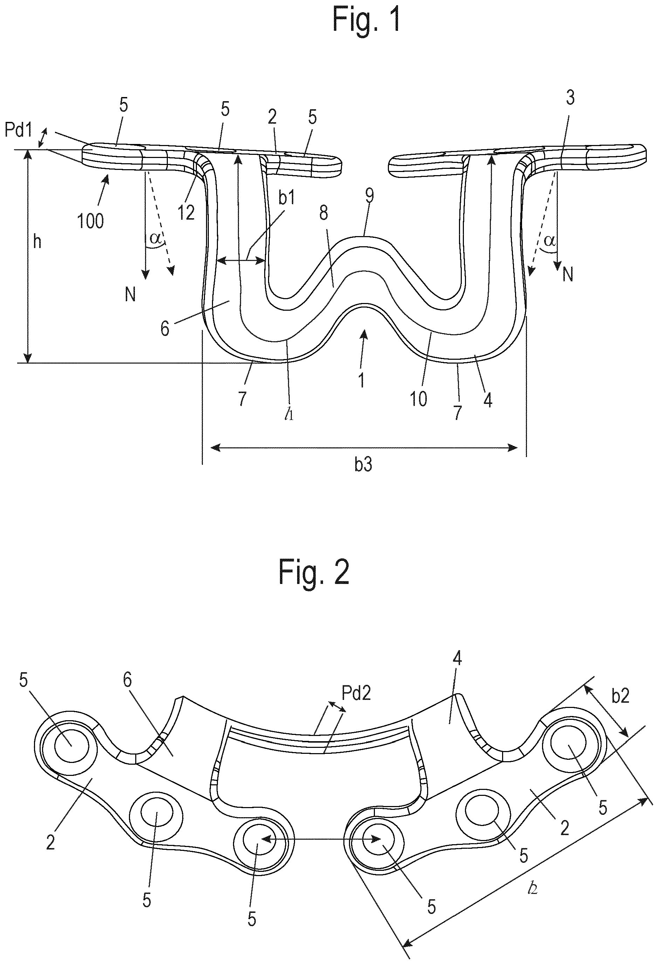

Die DE 102012 010 024 A1 offenbart einen Implantatträger, welcher allerdings nicht der gattungsgemäßen Ausgestaltung des Oberbegriffs des vorliegenden Erfindung entspricht. Ziel der Merkmale des Oberbegriffs ist die Definition eines Gegenstands mit welchem eine spezielle Kraftumlenkung in das Implantat bei einer Bewegung einhergeht. Die eingeleitete bewegungsbedingte Kraft wird dabei anders als bei der DE 26 03 087 B1 nicht senkrecht zur Fixationsplatte in das Verbindungssegment abgeleitet. Vielmehr wirken in der DE 10 2012 010 024 A1 durch die direkte und kürzeste Verbindung zweier Fixationsplatten, Zugkräfte auf ebendiese Fixationsplatten ein, wodurch es zur seitlichen Belastung der Verbindungselemente (Knochenschrauben und dergl.) kommt. Weiterhin besteht das offenbarte Konstrukt aus mindestens 13 Teile, die zusammengebaut werden und mittels eine Traverse Titanstab eine seitliche Verschiebung der zwei Knochenplatten erlauben. Die Bewegung findet nur in einer Ebene statt - die seitliche Ebene und das Ausmaß der Bewegung ist durch die Länge des Schlitzes in der Traverse definiert (Fig 1, no 20). Die Traverse gleitet ohne Widerstand.

Gleiches gilt für die US 2005/0165401 A1. Die Platte ist eine Platte zur Stabilisierung der Acetabulumfrakturen (Frakturen der Hüftpfanne) - also eine Fraktur im Bereich des Beckens - aber örtlich 10 cm von der Symphyse entfernt. Speziell für diese Platte ist eine zusätzliche Fläche in der Mitte der Platte dessen Zweck ist die Acetabulum-Fraktur abzustützen. Die Fläche stützt Lamina Quadrilateralis ab und verhindert die Protrusion/Medialisierung des Femurkopfes. Das Konstrukt verfolgt im Aufbau, Ausführung und Zweck ein anderes Ziel als die vorliegende Erfindung.

In der DE 10 2005 032 026 B3 wird eine Platte mit zwei Plattenabschnitten offenbart, die in verschiedenen Winkeln verbunden sind. Die Platte ist für Kieferchirurgie vorgesehen und verschiedenen Winkeln zwischen Plattenabschnitte sind die spezielle Geometrie des Kiefers angepasst. Figur 14a und 14b zeigt zwei Platten die mit einem U-förmigen Metallplatte verbunden sind. Der Zweck dieser Verbindung ist Nerven umfahren zu können, und dadurch eine Einklemmung des Nervs unter der Platte zu verhindern. Auch ist die Biegung des U-formigen Teils möglich und damit die Platte der Geometrie des Kiefers besser anpassen zu können wie aus den Absätzen [0066] und [0067] hervorgeht. Die Biegung ist somit "in plane" also entlang der Ebene der Auflagenflächen der Fixationselemente und kann daher keine axiale Verschiebung der Beckenknochen gegeneinander abfedern und stabilisieren.

Somit bildet die DE 26 03 087 B1 den einzigen bekannten gattungsgemäßen Stand der Technik, welcher die Krafteinbringung und -umlenkung in die gewünschte Richtung überträgt. Allerdings ist die Osteosyntheseplatte der gattungsgemäßen DE 26 03 087 B1 breitflächig ausgebildet und umschließt bzw. umgreift den Beckenrand und die Beckeninnenseite wie eine Manschette. Die Oberfläche der Platte ist insbesondere auf maximale Grösse gebracht damit eine hohe Steifigkeit der Platte erreicht wird. Auch werden Schrauben nah zu der Symphyse eingeschraubt so dass die Platte eine maximale Stabilität erreicht. Darüber hinaus liegt die Platte an der äusseren Fläche des Beckens - also zwischen Haut und Knochen.

Durch die breitflächige Ausbildung insbesondere im Bereich des Verbindungssegments werden allerdings die Fixationsplatten starr fixiert. Dies ist insofern problematisch als dass die verbundenen Beckenknochen infolge einer Bewegung selbst gegeneinander eine Bewegung ausführen, so dass es bei starrer Fixierung zu einem allmählichen Lösen der Verbindungsstellen der Osteosyntheseplatte mit dem Beckenknochen kommen kann.

Ausgehend von der vorgenannten Problemstellung ist es Aufgabe der vorliegenden Erfindung eine Osteosyntheseplatte bereitzustellen, welche eine Kraftumlenkung

ermöglicht und teilweise auch einer bewegungsbedingten Kraft durch elastische Verformung entgegenwirkt.

Die vorliegende Erfindung löst die vorgenannte Aufgabe durch eine Osteosyntheseplatte mit den Merkmalen des Anspruchs 1.

Es ist eine Grundidee der vorliegenden Erfindung eine Osteosyntheseplatte nicht starr, sondern federn und zugleich ergonomisch auszubilden. Der Grad einer Federung ist dem Fachmann als Federhärte bekannt und wird üblicherweise über eine sogenannte Federkonstante angegeben. Die Federkonstante ist dabei ein Wert, der einer Feder zugesprochen wird. Man verwendet die Federkonstante, um die Feder für den gewünschten Zweck auslegen und berechnen zu können. Die Federkonstante ist eine komplexe physikalische Größe und kann durch verschiedene Faktoren der Feder beeinflusst werden. Sie errechnet sich aus der Auslenkung der Feder mit der dazu notwendigen und resultierenden Kraft. (Siehe u.a. https:/Iwww.maschinenbau-wissen.de/skript3/mechanik/kinetik/114-federkonstante). Dem Fachmann ist eine Federkonstante somit nicht als ein zu erreichendes Ergebnis, sondern als physikalische Größe und somit als technisches Merkmal zur Beschreibung eines Federelements bekannt. Federkonstanten werden daher seit Langem auch in der Patentliteratur als technische Merkmale zur Beschreibung der Weichheit oder Härte eines Federelements benutzt. Beispiele für eine Federkonstante als geeignetes Merkmal zur Charakterisierung eines Federelements sind folgende erteilte EP-Patente:
In der EP 2 704 974 B1 (Anspruch 1) wurde eine Aufwickelvorrichtung mit einer Federkonstante zwischen 0,5 und 5 N/m charakterisiert.

In der EP 0 861 571 B1 (Anspruch 1) wurde ein Schaummaterial über seine dynamische Federkonstante von mindestens 4,4*10⁴ N/m charakterisiert.

In der EP 2 438 953 B1 (Anspruch 12) wurde die Feder eines zusammenziehbaren Atemschlauchs anhand seiner Federkonstante charakterisiert.

Es finden sich aber auch zahlreiche weitere Beispiele zur Nutzung der Federkonstante als technisches Merkmal in der Patentliteratur.

Eine erfindungsgemäße Osteosyntheseplatte sollte sich als Ersatz einer Synarthrose eignen. Dabei weist die Osteosyntheseplatte zwei Fixationsplattensegmente zur Festlegung der Osteosyntheseplattes an zwei Knochen eines Beckens auf.

Jedes der Fixationsplattensegmente definiert eine Auflagenfläche. Diese kann eben, gewölbt oder individuell auf den Patienten angepasst sein. Die Auflagefläche dient speziell im Anwendungsfall des Symphyseersatzes im Beckenbereich zur Auflage auf dem Beckenrand eines Beckenknochens, während das Verbindungselement gegenüber den Fixationsplattensegmenten derart hervorsteht, dass es sich zumindest bereichsweise entlang der Beckeninnenseite bzw. über einen Teilbereich der Beckeninnenseite, erstreckt und besonders bevorzugt daran anliegt.

Unabhängig von dem vorgenannten bevorzugten Anwendungsfall, weist jede Auflagenfläche, unabhängig von ihrer Form zumindest einen Normalenvektor auf, welcher senkrecht zu der Auflagenfläche steht.

Die Osteosyntheseplatte weist zudem ein Verbindungssegment auf, welches die beiden Fixationsplattensegmente miteinander verbindet und welches aus den Fixationsplattensegmenten hervorsteht. Während die Fixationsplattensegmente in Weiterführung ihrer Erstreckung zu einer theoretischen Verbindung der Fixationsplattensegmenten im kürzesten Abstand eine Verbindungsebene definieren, so liegt das Verbindungssegment vorzugsweise außerhalb dieser Verbindungsebene.

Dabei kann das Verbindungssegment randseitig am Fixationsplattensegment angeordnet sein und um einen Winkel von mehr als 45° gegenüber einer fiktiven Verlängerung der Auflagenfläche abgewinkelt, insbesondere abgebogen, sein. Diese abgewinkelte Abbiegung bezeichnet eine Ausrichtung gegenüber einer in-Plane (Ausrichtung in der Ebene der Auflagenfläche) bzw. einer Abbiegung gegenüber der vorgenannten fiktiven Verlängerung. Sie ist als eine Angabe eines Betrags zu verstehen und kann daher auch ein Negativwert für eine Winkelangabe im streng mathematischen Sinne darstellen.

Umgekehrt kann das Verbindungssegment vorteilhaft um weniger als 45° gegenüber dem Normalenvektor abweichen,
Die erfindungsgemäße Osteosyntheseplatte kann vorteilhaft aufgrund ihrer Elastizität die Krafteinwirkung auf die Verbindungsstelle zum Knochen auf längere Zeit verteilen, wodurch die Belastung auf die Verbindungsstelle abgedämpft wird.

Weitere vorteilhafte Ausgestaltungen sind u.a. Gegenstand der Unteransprüche.

Ausgehend vom Fixationsplattensegment kann in einer bevorzugten Ausführungsvariante randseitig daran eine mechanische Schnittstelle als Teil des Verbindungssegments angeordnet sein, welche als ein Bogen bzw. eine Biegung ausgebildet ist. Ausgehend nach dieser mechanischen Schnittstelle verläuft das Verbindungssegment in dem vorgenannten Winkel. Die mechanische Schnittstelle nimmt dabei bezogen auf die Gesamtlänge des mechanischen Verbindungssegments weniger als 10%, vorzugsweise weniger als 5% der Gesamtlänge ein. Die mechanische Schnittstelle kann im Rahmen der vorliegenden Erfindung vorzugsweise eine Schweißstelle oder eine Biegestelle sein.

In der weiteren Erstreckung verläuft das Verbindungssegment in einem Winkel von weniger als 45° gegenüber dem Normalenvektor. Das bedeutet, dass es auch Teilbereiche des Verbindungssegments geben kann welche einen Winkel von mehr als 45° gegenüber dem Verbindungssegment einnehmen. Allerdings ist dies bevorzugt lediglich, wie vorhergehend definiert, lediglich ein geringer Abschnitt des Verbindungssegments.

Um zusätzlich eine Kraftverminderung der einwirkenden Kräfte zu erreichen, ist das Verbindungssegment als Federelement ausgebildet. In dieser Funktion ist das Verbindungssegment derart ausgebildet, dass ein frei-schwingendes Fixationsplattensegment der Osteosyntheseplatte im einseitig fixierten Zustand gegenüber dem zweiten Fixationsplattensegment bei Aufwendung einer Kraft von 350 N aus einer Richtung parallel zum Normalenvektor um eine Distanz Δs von zumindest 1,5 mm, vorzugsweise 1,8 bis 5,0 mm, ausgelenkt wird. Die Auslenkung und die vorhergehende Definition ersetzt dabei die Angabe einer Federkonstante zur Charakterisierung des Verbindungssegments als Federelement. Die Kraftminderung bewirkt, dass die auf die Knochenschrauben einwirkenden Kräfte verringert sind.

Das Verbindungssegment kann vorteilhaft als ein Flachverbindungssegment ausgebildet sein mit einer mittleren Plattenstärke, welche zumindest 1,5 mal geringer ist, vorzugsweise 2-5 mal geringer ist, als die mittlere Breite des Flachsegments. Durch diese Ausgestaltung wird eine besondere mechanische Stabilität der Verbindung gewährleistet.

Die Länge des Verbindungssegments kann zumindest doppelt so lang sein wie der kürzeste Abstand der Kanten der beiden Fixationsplattensegmente, um dadurch eine hohe Flexibilität für eine Ausgleichsverformung der Osteosyntheseplatte zu erreichen.

Aus einem ähnlichen Grund ist es auch von Vorteil, wenn die Länge des Verbindungssegments zumindest fünfmal so lang ist wie die mittlere Breite des Verbindungssegments.

Eine besonders gute mechanische Stabilität in Kombination mit einer optimalen Federwirkung wurden beobachtet, sofern das Verbindungssegment eine "W" oder "U"-Form aufweist.

Bevorzugt steht das das Verbindungssegment um mehr als 45°, vorzugsweise zwischen 85-95°, gegenüber dem Fixationsplattensegment hervor. Besonders bevorzugt kann es sich dabei um eine Abbiegung gegenüber dem Fixationsplattensegment mit einem entsprechenden Biegebereich handeln.

Die Fixationsplatten können für eine stabile und zugleich kraftverteilende Verbindung zu einem Knochen zumindest zwei, vorzugsweise drei Bohrlöcher zur Durchführung jeweils eines mechanischen Verbindungsmittels, insbesondere einer Knochenschraube aufweisen.

Bevorzugt ist die Osteosyntheseplatte einstückig ausgebildet, um eine optimale Kraftumleitung durch das Material zu ermöglichen. Für eine Federwirkung und zur Aufteilung von Materialspannungen ist es günstig, wenn die gesamte Osteosyntheseplatte aus einem duktilen Material, besonders bevorzugt aus einem Titan und/oder einem Edelstahl und/oder einer Metalllegierung umfassend Titan und/oder Eisen besteht.

Um eine Verträglichkeit zu verbessern ist es von Vorteil, wenn die Osteosyntheseplatte zumindest abschnittsweise eine Beschichtung, insbesondere aus einem resorbierbaren Material oder mit einem entzündungshemmenden Wirkstoff, aufweist. Dies kann insbesondere im Bereich der Auflageflächen sein.

Fertigungstechnisch ist es günstig, wenn die Fixationsplattensegmente und das Verbindungssegment die gleiche Plattenstärke aufweisen. Geringe Abweichungen von 10% der Plattenstärke oder weniger sind dabei zu vernachlässigen. Darüber hinaus werden dadurch keine zusätzlichen mechanischen Schwachstellen geschaffen.

Die Fixationsplattensegmente weisen eine Längserstreckung und eine Quererstreckung senkrecht dazu auf, wobei das Verbindungssegment randseitig und mittig bezogen auf die Längserstreckung angeordnet ist. Dadurch ist die Kraftübertragung auf alle Bohrlöcher des Fixationsplattensegments bei einer Verformung gleichmäßig verteilt.

Bevorzugt ist eine Schnittstelle zwischen jeweils einem Fixationsplattensegment und dem Verbindungssegment als eine Biegung ausgebildet, vorzugsweise mit einem Biegeradius, welcher zumindest größer, besonders bevorzugt zumindest doppelt so groß, wie die mittlere Plattenstärke des Verbindungssegments, so dass eine Kraftumlenkung optimiert erfolgt.

Für eine zusätzliche Verformung in eine zweite Dimension kann das Verbindungssegment gewölbt und zu den Fixationspunkten der Fixationsplattensegmente hin konvex ausgebildet sein. Dadurch wird eine noch bessere Kraftminderung erreicht.

Wie aus sich bereits aus dem Kontext der Beschreibung ergibt ist eine Osteosyntheseplatte im Rahmen der vorliegenden Erfindung nicht als ausschließlich ebenes Objekt zu verstehen, sondern es kann eines oder mehrere Teilsegmente, beispielsweise ein abgebogenes Verbindungssegment, aufweisen, welche insbesondere winkelig aus einer Plattenebene, vorzugsweise auch zu mehr als 45°, insbesondere senkrecht, hervorstehen können.

Weitere Vorteile, Merkmale und Einzelheiten einer erfindungsgemäßen Osteosyntheseplatte ergeben sich aus der nachfolgenden Beschreibung, in der ein Ausführungsbeispiel der Erfindung anhand der beiliegenden Zeichnungen näher erläutert wird. Der Fachmann wird die in den Zeichnungen, der Beschreibung und den Ansprüchen in Kombination offenbarten Merkmale zweckmäßigerweise auch einzeln betrachten und zu sinnvollen weiteren Kombinationen zusammenfassen. Insbesondere gibt es eine Vielzahl von Möglichkeiten, diese im Rahmen der vorliegenden Erfindung abzuwandeln und weiterzubilden. Es zeigen:
- Fig. 1: eine Ansicht einer Ausführungsvariante einer erfindungsgemäßen Osteosyntheseplattes in die koronare Ebene bzw. antero-posteriore Ebene;
- Fig. 2: eine Ansicht auf die erfindungsgemäße Osteosyntheseplatte in axialer Ebene;
- Fig. 3: eine Kraftverteilungsanzeige in der Perspektivansicht auf die erfindungsgemäße Osteosyntheseplatte;
- Fig. 4: eine schematische Darstellung der Osteosyntheseplattes im montierten Zustand in einer Anwendung als Ersatz einer Symphyse; und
- Fig 5: eine Ansicht auf die erfindungsgemäße Osteosyntheseplatte der Fig. 1-4 in sagittaler Ebene.

Das in den Fig. 1-5 dargestellte Ausführungsbeispiel zeigt eine Osteosyntheseplatte 1 zur Fixierung eines Beckens insbesondere aufgrund eines traumatisch-bedingten Ruptur der Symphyse.

Eine derartige Verletzung sorgt für eine Instabilität des Beckenrings und muss auf chirurgische Weise stabilisiert werden. Bekannte Knochenplatten sind leistenförmig gerade oder winklig ausgebildet.

Die erfindungsgemäße Osteosyntheseplatte 1 weist ein erstes und eins zweite Fixationsplattensegment 2 und 3 auf, sowie ein Flachverbindungssegment 4 auf, welche zwischen den beiden Fixationsplatten 2, 3 angeordnet ist und diese miteinander verbindet. Das Flachverbindungssegment 4 weist eine mittlere Plattendicke Pd2 von vorzugsweise zumindest 2 mm, vorzugsweise zwischen 2,5 bis 5 mm, insbesondere 3 mm +/- 0,2 mm, auf. Besonders bevorzugt ist die Plattendicke Pd2 des Flachverbindungssegments 4 im Wesentlichen, also über mehr als 90% der Länge l1 des Flachverbindungssegments 4, einheitlich.

Das Flachverbindungssegment 4 weist vorzugsweise mittlere Breite b1 von vorzugsweise zumindest 5 mm, vorzugsweise zwischen 7 - 15 mm, insbesondere 9 mm +/-0.8 mm, auf. Besonders bevorzugt ist die Plattendicke Pd2 des Fachverbindungssegments 4 im Wesentlichen, also über mehr als 90% der Länge l1 des Flachverbindungssegments 4, einheitlich.

Die erste und die zweite Fixationsplatte 2 und 3 definiert jeweils eine erste und eine zweite Auflagenfläche 100 mit einem Normalenvektor N. Die beiden Auflageflächen 100 können zueinander parallel oder auf einer gemeinsamen Ebene liegen. Allerdings können die beiden Auflagenflächen 100 auch gebogen ausgebildet und/oder winklig zueinander angeordnet sein.

Ausgehend von einem der beiden Normalenvektoren N, oder einem parallel zu diesem Normalenvektor verlaufenden Vektor, steht das Flachverbindungssegment 4 mit einem Winkel α von weniger als 45°, insbesondere in einem Winkel von weniger als 5°, gegenüber dem besagten jeweiligen Normalenvektor oder dem parallelen Vektor aus der jeweiligen Fixationsplatte 2 oder 3 hervor. In Fig. 1 steht das Flachverbindungssegment 4 um diesen Winkel aus der Zeichnungsebene hervor. Das Hervorstehen erfolgt derart, dass ein and den Fixationsplattensegmenten angeordnetes randseitiges Materialsegment, das Flachverbindungssegment, gegenüber den Fixationsplattensegmenten abgebogen ist. Die Biegung wird nachfolgend auch als mechanische Schnittstelle 12 bezeichnet und ist ein Abschnitt des Flachverbindungssegments 4.

Jede der Fixationsplatte 2 und 3 verfügt zumindest über einen, vorzugsweise zumindest drei Fixationspunkte 5 zur Fixierung der besagten Fixationsplatte an einem Knochen, insbesondere einem Beckenknochen. Die Fixationspunkte 5 können als Bohrlöcher ausgebildet sein, durch welche über entsprechende Schrauben 6, z.B. Knochenschrauben, eine Fixierung mit dem Knochen gelingt.

Das Flachverbindungssegment 4 ist dabei vorzugsweise derart ausgebildet, dass es eine größere Länge l₁ aufweist, als der kürzeste direkte Abstand r zwischen zwei an geringsten beabstandete Fixationspunkte 5 zwischen den zwei Fixationsplattensegmenten 2 und 3. Mit anderen Worten, der kürzeste Abstand r je von einem Fixationspunkt 5 der erste Fixationsplatte 2 zu einem Fixationspunkt 5 der zweiten Fixationsplatte 3 wird ermittelt. Die Länge l₁ des Flachverbindungssegments 4 ist größer als dieser Abstand r.

Die Osteosyntheseplatte 1 ist vorzugsweise einstückig ausgebildet und besteht aus einem duktilen Werkstoff, vorzugsweise einem Metall, insbesondere aus Titan und/oder Edelstahl oder einer Legierung mit zumindest einem der beiden Metalle Titan und/oder Eisen.

Die Fixationsplattensegmente 2, 3 in der Variante der Fig. 1-3 ist als Dreilochplatte ausgebildet, das heißt sie weist drei Bohrungen zur Durchführung von Knochenschrauben 6 und zur Fixation der Osteosyntheseplatte an den zwei Beckenknochen 13, welche durch einen kompletten oder nicht-kompletten Ruptur der Symphyse voneinander getrennt wurden. Die Bohrungen sind in Längsrichtung des Fixationsplattensegments 2 oder 3 hintereinander angeordnet. Die Bohrungen entsprechen den vorgenannten Fixationspunkten 5. Die Bohrungen können so gewählt werden, dass sie zur Durchführung von Knochenschrauben zwischen 2-5 mm Durchmesser, vorzugsweise 3-4 mm, bestimmt sind.

Das Flachverbindungssegment 4 geht randseitig auf Höhe der mittleren Bohrung von dem Fixationsplattensegment 2, 3 ab und ist in einem im Wesentlichen senkrechten Winkel von dem Fixationsplattensegment 2, 3 abgewinkelt bzw. gebogen.

Das Flachverbindungssegment 4 weist ausgehend von dem Fixationsplattensegment 2 einen ersten Bereich 6 auf mit einem ersten negativen Anstieg bis zu einen ersten Minimum 7, wobei der negative Anstieg von dem Fixationsplattensegment weggerichtet ist.

Sodann weist das Flachverbindungssegment 4 einen zweiten Bereich 8 mit einem ersten positiven Anstieg auf, welcher bis zu einem ersten Maximum 9 verläuft. Diese Länge dieses zweiten Bereichs 8 ist geringer, vorzugsweise um mehr als 30% geringer als die Länge des ersten Bereichs. Der positive Anstieg ist dabei zum Fixationsplattensegment 2 hin gerichtet. Das Maximum 9 weist somit einen Abstand zum ersten und zweiten Fixationsplattensegment 2 und 3 und zu deren Verbindungsachse auf.

Sodann weist das Flachverbindungssegment 4 einen dritten Bereich 10 mit einem zweiten negativen Anstieg auf, welcher wiederum bis zu einem zweiten Minimum 11 verläuft. Der erste und der dritte Bereich 6 und 7 sind dabei im Wesentlichen gleich lang. Die Minima 7 und 11 sind im entspannten Zustand der Osteosyntheseplatte im Wesentlichen auf gleicher Höhe. Das Maximum 9 ist im gleichen Abstand zu den beiden Minima 7 und 11 angeordnet. Die Form des Flachverbindungssegments 4 entspricht der eines "W".

Wie bereits zuvor erläutert, jedoch nicht in den Figuren dargestellt, kann das Flachverbindungssegment in einer alternativen Ausführungsvariante auch die Form eines "U" aufweisen.

Die maximale Breite b₃ des "W"'s entspricht dabei vorzugweise zumindest 30 mm, besonders bevorzugt zwischen 35-60 mm, besonders bevorzugt 45 mm +/- 5 mm. Die maximale Höhe h des "W"'s, bzw. die Länge des ersten Bereichs 6, gemessen von der Oberfläche am Fixationsplattensegment 2 an, beträgt vorzugsweise zumindest 15 mm, vorzugsweise 20-45 mm, besonders bevorzugt 30 mm +/- 3 mm.

Allgemein formuliert ist in einer bevorzugten Ausführungsvariante der Erfindung die Breite "W" des Flachverbindungssegments 4 größer als dessen Höhe "H".

Die Form des Flachverbindungssegments 4 ist zudem konvex, bezogen auf die Position der Fixationsplattensegmente 2 und 3.

Die Länge l₂ des Fixationsplattensegments 2 beträgt vorzugsweise zwischen 25-45 mm, vorzugsweise 40 mm +/- 4 mm. Die mittlere Breite des Fixationsplattensegments b₂ über seine gesamte Länge beträgt vorzugsweise zwischen 6-13 mm, vorzugsweise 10 mm +/- 1mm.

Der Abstand r der Fixationsplattensegmente 2, 3 von Rand zu Rand ist vorzugsweise weniger als die Längserstreckung eines Fixationsplattensegments. Er kann vorzugsweise zwischen 6-10 mm, vorzugsweise 8 mm +/- 0,5 mm, betragen. Der Abstand der Fixationsplattensegmente 2, 3 kann allerdings unter Verformung und Ausbildung von Rückstellkräften variieren. Der vorgenannte Abstand bezieht sich auf eine Osteosyntheseplatte im ungespannten Zustand und unmontierten Zustand. Im unmontierten Zustand ist die Osteosyntheseplatte noch nicht an einem oder mehreren Knochen festgelegt.

Die mittlere Plattendicke Pd1 der Fixationsplattensegmente 2, 3 kann der mittleren Plattendicke des Flachverbindungssegments von Plattendicke von zumindest 2 mm, vorzugsweise zwischen 2,5 bis 5 mm, insbesondere 3 mm +/- 0,2 mm, entsprechen.

Alternativ zur bevorzugten Variante der W-Form der Osteosyntheseplatte 1 sind auch anderen Formen, wie z.B. eine U-Form oder eine V-Form vorstellbar. Die vorgenannten Bezeichnungen wie mittlere Breite oder mittlere Plattendicke impliziert, dass eine Schwankung im Verlauf des jeweiligen Segments auftreten kann und dass im Fall einer solchen Schwankung die Ermittlung eines Mittelwertes mit einer Messung der Werte an unterschiedlichen Positionen des jeweiligen Segments erforderlich ist. Die Angaben wie Länge, Breite usw. beziehen sich stets auf eine Osteosyntheseplatte im unmontierten und unbelasteten bzw. unverformten Zustand.

Aus den vorgenannten Werten lässt sich ableiten, dass die Breite des Flachverbindungssegments 4 zumindest 3-mal so groß, vorzugsweise 4-8 mal so groß, ist wie der Abstand der beiden Fixationsplattensegmente 2, 3 von Rand zu Rand.

Die Osteosyntheseplatte wirkt aufgrund seiner konstruktiven Ausgestaltung als federndes Verbindungsteil, welches einen Bewegungsausglich zwischen den Beckenknochen herstellt und zugleich hinreichend mechanische Festigkeit besitzt, um eine Aufweitung des Abstands der Beckenknochen im Bereich der Symphyse zu verhindern.

Die Federwirkung ist für diesen Bereich optimiert, so dass die Osteosyntheseplatte die Rückstell- und Zugkräfte einer Symphyse ersetzen kann. Hierzu wurden Tests durchgeführt.

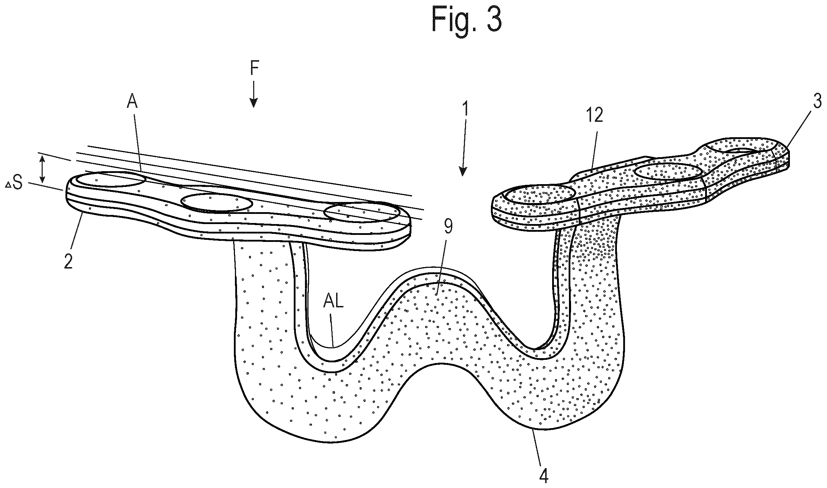

Im Rahmen des Tests wurde eines der beiden Fixationsplattensegmente (rechter Schenkel) fixiert, insbesondere verschraubt, und das zweite Fixationsplattensegment (linker Schenkel) frei schwingend war. Die Situation wird in Fig. 3 wiedergegeben.

Erfolgt nun eine Druckkraft F in Richtung des Normalenvektors auf die der Auflagenfläche 100 entgegengesetzten Oberfläche des Fixationsplattensegments 2 und parallel zu diesem Vektor, so verformt sich die Osteosyntheseplatte unter Ausbildung einer Rückstellkraft. Bei Entfernen der Druckkraft kehrt die Osteosyntheseplatte 1 in ihre Ausgangsstellung AS zurück.

Sodann wurden zur Bewegungssimulation 1200 Vollzyklen über 250 Sekunden auf das frei schwingende Fixationsplattensegment angewandt. Ein Vollzyklus besteht aus der Anwendung einer Druckkraft z.B. durch eine Druckstange, auf das Fixationsplattensegment bei einer definierten Kraft und aus der Rückkehr der Osteosyntheseplatte in seine Ausgangsstellung beim Entfernen der Druckkraft.

Die durchschnittliche applizierte Druckkraft betrug 351,5 N und führte zu einer Verformung Δs von 2,885 mm. Diese Verformung entspricht der Höhendifferenz des freischwingenden Fixationsplattensegments gegenüber seiner Ausgangslage. Aus Fig. 3 sind in schraffierter Darstellung die unterschiedliche Kraftverteilung innerhalb der Osteosyntheseplatte zu erkennen. Eine plastische Verformung konnte dabei nicht beobachtet werden, da es keine Varianz der Ausgangslage gab.

In einem zweiten Versuch wurden sodann 9999 Vollzyklen durchgeführt mit einer durchschnittlichen Druckkraft F von 357,6 N. Die Auslenkung bzw. Verformung Δs des frei schwingenden Fixationsplattensegments gegenüber seiner Ausgangslage bzw. Ausgangsstellung AS betrug 2,945 mm. In diesem Fall konnte eine geringfügige vernachlässigbare Verformung von 0,061 mm beobachtet werden.

Somit kann die Osteosyntheseplatte bei Einwirkungen von 10000 Vollzyklen bei einer Kraft von etwa 350 N als formstabil bezeichnet werden.

Experimente mit gesunden Probanden haben zudem gezeigt, dass die Symphyse bei dem Gangzyklus mit 170 N alternierend belastet wird und beim Rennen Maximalbelastungen bei erwachsenen bis 370 N entstehen und zu einer Bewegung von etwa 2-3 mm führt. Diese Kräfte können ideal auch durch die erfindungsgemäße Osteosyntheseplatte aufgenommen und entsprechend abgefedert werden.

Die Verformung Δs der freischwingenden Fixationsplattensegments der einseitig fixierten Osteosyntheseplatte bei Ausübung einer Kraft auf dieses Fixationsplattensegment in Richtung des Normalenvektors beträgt vorzugsweise zwischen 1,8 bis 5,0 mm bei Aufwendung einer Kraft von 350N.

Aus der Fig. 3 erkennt man eine Kraftverteilung bei einer Kraftapplikation auf das frei schwingende Fixationsplattensegment 2. Die Dichte der Punktierung gibt den Umfang der Materialspannungen in Antwort auf die Krafteinwirkung an. Wie man erkennt ist die Krafteinwirkung im Bereich der Schnittstelle 12 und dem fixierten Fixationsplattensegment am Größten. Eine Materialspannung in diesem Bereich führt zu einer axial-gerichteten Kraft, also auf die nicht-dargestellten Schraubenköpfe und nicht als seitlich gerichtete Kraft entlang der Ebene des Fixationsplattensegments, welche für allmähliche Aufweitung und Lockerung der Verbindung zwischen Schraube und Knochen führen würde.

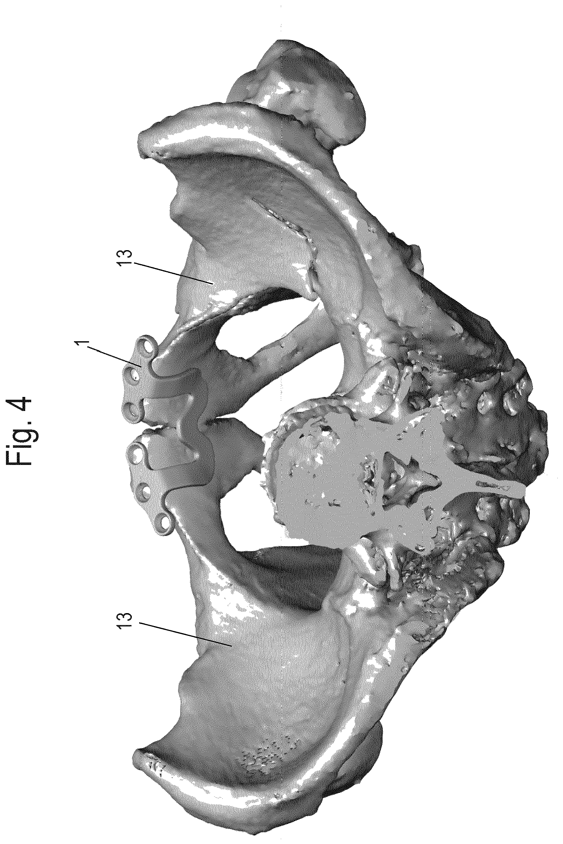

Das Beispiel der Fig. 1-5 bezieht sich vornehmlich auf eine Osteosyntheseplatte als Ersatz der fibrokartilaginösen Verbindung zwischen den Schambeinästen bei einer Ruptur der Symphyse. Dieser Anwendungsfall wird in Fig. 4 wiedergegeben.

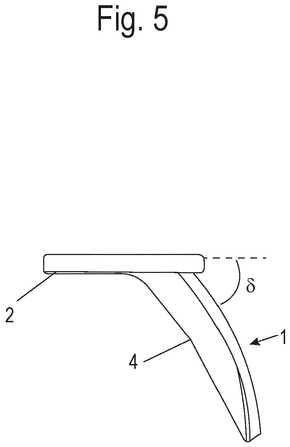

Wie aus Fig. 5 erkennbar, kann der Winkel zwischen Auflagefläche und Verbindungssegment beispielsweise 130° betragen oder, anders ausgedrückt, um einen Winkel δ von 50° gegenüber den Fixationsplattensegmenten hervorstehen.

Die Verbindung der erfinungsgemäßen Osteosyntheseplatte erlaubt insbesondere eine kontrollierte Bewegung zwischen den Segmenten durch eine gezielte Dimensionierung des Verbindungsegmentes.

Die zwei Fixationsplattensegmente und das Verbindungssegment zwischen den besagten Fixationsplattensegmenten sind Teil einer einstückigen Osteosyntheseplatte, insbesondere eines Stücks Metall. Es gibt keine Sammlungen oder Scharnieren. Besonders bevorzugt ist die einstückige Osteosyhtheseplatte monolithisch, also ohne Verbindungsnahten z.B. Schweißnahten oder dergl. augebaut.

Das Verbindungssegment zwischen den zwei Fixationsplattensegmenten ist durch die anatomischen Ansprüche des Beckens zwischen 30° und 80° im Vergleich zu den zwei Platten in der Seitenebene angewinkelt, und zwischen 0° und 30° im Vergleich zur Längsachse jeder Platte angewinkelt.

Insgesamt wird ein Implantat als Osteosyntheseplatte mit zwei Plattensegmenten bereitgestelt die mit einem flexiblen Verbindungssegment verbunden sind, die eine kontrollierte Bewegung zwischen den zwei Platten erlaubt.

### Bezugszeichen

- 1: Osteosyntheseplatte
- 2: Fixationsplattensegment
- 3: Fixationsplattensegment
- 4: Flachverbindungssegment
- 5: Fixationspunkt
- 6: erster Bereich
- 7: erstes Minimum
- 8: zweiter Bereich
- 9: erstes Maximum
- 10: dritter Bereich
- 11: zweites Minimum
- 12: Schnittstelle / Biegung
- 13: Beckenknochen

- 100: Auflagenfläche

- Pd1: Plattendicke (Fixationsplattensegment)
- Pd2: Plattendicke (Flachverbindungssegment)
- N: Normalenvektor

- F: Kraftrichtung
- Δs: Verformungsweg
- l₁: Gesamtlänge (Flachverbindungssegment)
- b: Breite (Flachverbindungssegment)
- α: Winkel
- δ: Winkel

- r: kürzester Abstand zweier Fixationspunkte
- bₘₐₓ: maximale Breite des Flachverbindungssegments
- h: Höhe des Flachverbindungssegments
- l₂: Länge des Fixationsplattensegments
- b₂: Breite des Fixationsplattensegments
- AS: Ausgangsstellung

## Patentansprüche

1. Osteosyntheseplatte (1) als Ersatz einer Synarthrose in Form einer Symphyse, wobei die Osteosyntheseplatte (1) zwei Fixationsplattensegmente (2, 3) zur Festlegung der Osteosyntheseplatte (1) an zwei Knochen aufweist,
wobei die Osteosyntheseplatte (1) ein Verbindungssegment aufweist, welches die beiden Fixationsplattensegmente (2, 3) miteinander verbindet,
wobei das Verbindungssegment aus den Fixationsplattensegmenten hervorsteht
wobei jedes der Fixationsplattensegmente (2, 3) eine Auflagenfläche (100) zur Auflage des Fixationsplattensegments (2, 3) an einem Beckenrand definiert und wobei das Verbindungssegment ausgebildet ist zur Erstreckung entlang der Beckeninnenseite,
**dadurch gekennzeichnet, dass** das Verbindungssegment derart ausgebildet ist, dass ein frei-schwingendes Fixationsplattensegment (2) der Osteosyntheseplatte (1) im einseitig fixierten Zustand der Osteosyntheseplatte (1) gegenüber dem zweiten Fixationsplattensegment (3) bei Aufwendung einer Kraft von 350 N aus einer Richtung parallel zum Normalenvektor (N) der Auflagenfläche (100) des Fixationsplattensegments (2) um eine Distanz Δs von zumindest 1,5 mm, vorzugsweise 1,8 bis 5,0 mm, ausgelenkt wird.

2. Osteosyntheseplatte (1) nach Anspruch 1, **dadurch gekennzeichnet, dass** die Auflagenfläche (100) einen Normalenvektor (N) aufweist, welcher senkrecht zu der Auflagenfläche (100) steht, und wobei das Verbindungssegment zumindest bereichsweise um einen Winkel (α) von weniger als 45° gegenüber dem Normalenvektor (N) der Auflagenfläche (100) oder einem dazu parallel-verschobenen Vektor gegenüber dem Fixationsplattensegment (2, 3) abgewinkelt ist.

3. Osteosyntheseplatte nach einem der vorhergehenden Ansprüche 1 oder 2, **dadurch gekennzeichnet, dass** das Verbindungssegment als ein Flachverbindungssegment (4) ausgebildet ist mit einer mittleren Plattenstärke (Pd2), welche zumindest 1,5 mal geringer ist, vorzugsweise 2-5 mal geringer ist, als die mittlere Breite (b1) des Flachverbindungssegments (4).

4. Osteosyntheseplatte nach einem der vorhergehenden Ansprüche, **dadurch gekennzeichnet, dass** die Länge (11) des Verbindungssegments zumindest doppelt so lang ist wie der kürzeste Abstand r der Ränder der beiden Fixationsplattensegmente (2, 3).

5. Osteosyntheseplatte nach einem der vorhergehenden Ansprüche, **dadurch gekennzeichnet, dass** die Länge (11) des Verbindungssegments zumindest fünfmal so lang ist wie die mittlere Breite (b1) des Verbindungssegments.

6. Osteosyntheseplatte nach einem der vorhergehenden Ansprüche, **dadurch gekennzeichnet, dass** das Verbindungssegment eine "W" oder "U"-Form aufweist.

7. Osteosyntheseplatte nach einem der vorhergehenden Ansprüche, **dadurch gekennzeichnet, dass** das Verbindungssegment um einem Winkel (δ) von mehr als 45°, vorzugsweise 85-95°, gegenüber einer fiktiven Verlängerung der Auflagenfläche (100) eines jedem der Fixationsplattensegmente (2, 3), gegenüber dieser Verlängerung abgebogen ist.

8. Osteosyntheseplatte nach einem der vorhergehenden Ansprüche, **dadurch gekennzeichnet, dass** die Fixationsplatten (2, 3) zumindest zwei, vorzugsweise drei Bohrlöcher zur Durchführung jeweils eines mechanischen Verbindungsmittels, insbesondere einer Knochenschraube aufweisen.

9. Osteosyntheseplatte nach einem der vorhergehenden Ansprüche, **dadurch gekennzeichnet, dass** die Osteosyntheseplatte (1) einstückig ausgebildet ist und vorzugsweise aus einem duktilen Material, besonders bevorzugt aus einem Titan und/oder einem Edelstahl und/oder einer Metalllegierung umfassend Titan und/oder Eisen besteht.

10. Osteosyntheseplatte nach einem der vorhergehenden Ansprüche, **dadurch gekennzeichnet, dass** die Fixationsplattensegmente (2, 3) und das Verbindungssegment die gleiche Plattenstärke (Pd1, Pd2) aufweisen.

11. Osteosyntheseplatte nach einem der vorhergehenden Ansprüche, **dadurch gekennzeichnet, dass** die Fixationsplattensegmente (2, 3) eine Längserstreckung (l2) und eine Quererstreckung (b2) senkrecht dazu aufweisen, wobei das Verbindungssegment randseitig und mittig bezogen auf die Längserstreckung (l2) angeordnet ist.

12. Osteosyntheseplatte nach einem der vorhergehenden Ansprüche, **dadurch gekennzeichnet, dass** eine Schnittstelle (12) zwischen jeweils einem Fixationsplattensegment (2 oder 3) und dem Verbindungssegment als eine Biegung ausgebildet ist, vorzugsweise mit einem Biegeradius, welcher zumindest größer, besonders bevorzugt zumindest doppelt so groß, wie die mittlere Plattenstärke (Pd2) des Verbindungssegments.

13. Osteosyntheseplatte nach einem der vorhergehenden Ansprüche, **dadurch gekennzeichnet, dass** das Verbindungssegment gewölbt und zu Fixationspunkten (5) der Fixationsplattensegmente (2, 3) hin konvex ausgebildet ist.

## Claims

1. Osteosynthesis plate (1) for replacing a synarthrosis in the form of a symphysis, wherein the osteosynthesis plate (1) comprises two fixation plate segments (2, 3) for securing said osteosynthesis plate (1) to two bones,
wherein osteosynthesis plate (1) comprises a connecting segment which interconnects the two fixation plate segments (2, 3),
wherein the connecting segment protrudes from the fixation plate segments, wherein each of the fixation plate segments (2, 3) defines a support surface (100) for supporting the fixation plate segment (2, 3) on a pelvic edge, and wherein the connecting segment is designed to extend along the inner side of the pelvis, **characterized in that** the connecting segment is designed in such a way that a free-swinging fixation plate segment (2) of the osteosynthesis plate (1) in the unilaterally fixed state of the osteosynthesis plate (1) is deflected relative to the second fixation plate segment (3) upon application of a force of 350 N from a direction parallel to the normal vector (N) of the support surface (100) of the fixation plate segment (2) by a distance Δs of at least 1.5 mm, preferably 1.8 to 5.0 mm.

2. Osteosynthesis plate (1) according to claim 1, **characterized in that** the support surface (100) has a normal vector (N) which is perpendicular to the support surface (100), and wherein the connecting segment is angled at least in regions by an angle (□) of less than 45° relative to the normal vector (N) of the support surface (100) or a vector displaced parallel thereto relative to the fixation plate segment (2, 3).

3. Osteosynthesis plate according to one of the preceding claims 1 or 2, **characterized in that** the connecting segment is formed as a flat connecting segment (4) with an average plate thickness (Pd2) which is at least 1.5 times less, preferably 2-5 times less, than the average width (b1) of the flat connecting segment (4).

4. Osteosynthesis plate according to one of the preceding claims, **characterized in that** the length (11) of the connecting segment is at least twice as long as the shortest distance r between the edges of the two fixation plate segments (2, 3).

5. Osteosynthesis plate according to one of the preceding claims, **characterized in that** the length (11) of the connecting segment is at least five times as long as the average width (b1) of the connecting segment.

6. Osteosynthesis plate according to one of the preceding claims, **characterized in that** the connecting segment has a "W' or "U" shape.

7. Osteosynthesis plate according to one of the preceding claims, **characterized in that** the connecting segment is bent by an angle (δ) of more than 45°, preferably 85-95°, relative to a ficticious extension of the support surface (100) of each of the fixation plate segments (2, 3), with respect to this extension.

8. Osteosynthesis plate according to one of the preceding claims, **characterized in that** the fixation plates (2, 3) have at least two, preferably three, drilled holes for the passage of a respective mechanical connecting means, in particular a bone screw.

9. Osteosynthesis plate according to one of the preceding claims, **characterized in that** the osteosynthesis plate (1) is formed in one piece and preferably consists of a ductile material, particularly preferably of a titanium and/or a stainless steel and/or a metal alloy comprising titanium and/or iron.

10. Osteosynthesis plate according to one of the preceding claims, **characterized in that** the fixation plate segments (2, 3) and the connecting segment have the same plate thickness (Pd1, Pd2).

11. Osteosynthesis plate according to one of the preceding claims, **characterized in that** the fixation plate segments (2, 3) have a longitudinal extension (l2) and a transverse extension (b2) perpendicular thereto, wherein the connecting segment is arranged at the edge and centrally relative to the longitudinal extension (l2).

12. Osteosynthesis plate according to one of the preceding claims, **characterized in that** an interface (12) between a respective fixation plate segment (2 or 3) and the connecting segment is formed as a bend, preferably with a bending radius which is at least larger, particularly preferably at least twice as large, as the average plate thickness (Pd2) of the connecting segment.

13. Osteosynthesis plate according to one of the preceding claims, **characterized in that** the connecting segment is curved and convex towards fixation points (5) of the fixation plate segments (2, 3).

## Revendications

1. Plaque d'ostéosynthèse (1) pour le remplacement d'une synarthrose formant une symphyse, laquelle plaque d'ostéosynthèse (1) comporte deux segments de plaque de fixation (2, 3) pour la fixation de la plaque d'ostéosynthèse (1) sur deux os, laquelle plaque d'ostéosynthèse (1) comporte un segment de liaison qui relie les deux segments de plaque de fixation (2, 3) l'un à l'autre,
le segment de liaison dépassant des segments de plaque de fixation,
chacun des segments de plaque de fixation (2, 3) définissant une surface d'application (100) pour l'application du segment de plaque de fixation (2, 3) sur un bord du bassin et le segment de liaison étant conçu pour s'étendre le long de la face intérieure du bassin,
**caractérisée en ce que** le segment de liaison est conçu de telle manière qu'un segment de plaque de fixation (2) de la plaque d'ostéosynthèse (1) oscillant librement quand la plaque d'ostéosynthèse (1) est fixée d'un seul côté est dévié par rapport au deuxième segment de plaque de fixation (3) d'une distance Δs d'au moins 1,5 mm, de préférence de 1,8 à 5,0 mm, lorsqu'une force de 350 N est exercée depuis une direction parallèle au vecteur normal (N) de la plaque d'application (100) du segment de plaque de fixation (2).

2. Plaque d'ostéosynthèse (1) selon la revendication 1, **caractérisée en ce que** la plaque d'application (100) présente un vecteur normal (N) qui est perpendiculaire à la plaque d'application (100), et dans laquelle le segment de liaison est angulé au moins par zones par rapport au segment de plaque de fixation (2, 3) d'un angle (α) de moins de 45° par rapport au vecteur normal (N) de la plaque d'application (100) ou à un vecteur décalé parallèlement à celui-ci.

3. Plaque d'ostéosynthèse selon l'une des revendications 1 ou 2, **caractérisée en ce que** le segment de liaison est conformé comme un segment de liaison plat (4) ayant une épaisseur moyenne de plaque (Pd2) au moins 1,5 fois plus petite, de préférence 2,5 fois plus petite, que la largeur moyenne (b1) du segment de liaison plat (4).

4. Plaque d'ostéosynthèse selon l'une des revendications précédentes, **caractérisée en ce que** la longueur (11) du segment de liaison est au moins deux fois plus grande que la distance (r) la plus courte entre les bords des deux segments de plaque de fixation (2, 3).

5. Plaque d'ostéosynthèse selon l'une des revendications précédentes, **caractérisée en ce que** la longueur (11) du segment de liaison est au moins cinq fois plus grande que la largeur moyenne (b1) du segment de liaison.

6. Plaque d'ostéosynthèse selon l'une des revendications précédentes, **caractérisée en ce que** le segment de liaison a la forme d'un « W » ou d'un « U ».

7. Plaque d'ostéosynthèse selon l'une des revendications précédentes, **caractérisée en ce que** le segment de liaison est plié selon un angle (δ) de plus de 45°, de préférence de 85 à 95°, par rapport à un prolongement imaginaire de la plaque d'application (100) de chacun des segments de plaque de fixation (2, 3) par rapport à ce prolongement.

8. Plaque d'ostéosynthèse selon l'une des revendications précédentes, **caractérisée en ce que** les plaques de fixation (2, 3) comportent au moins deux, de préférence trois, trous percés pour le passage d'un moyen d'assemblage mécanique, en particulier d'une vis à os, dans chacun.

9. Plaque d'ostéosynthèse selon l'une des revendications précédentes, **caractérisée en ce que** la plaque d'ostéosynthèse (1) est formée d'une seule pièce et se compose de préférence d'un matériau ductile, en particulier d'un titane et/ou d'un acier inoxydable et/ou d'un alliage métallique contenant du titane et/ou du fer.

10. Plaque d'ostéosynthèse selon l'une des revendications précédentes, **caractérisée en ce que** les segments de plaque de fixation (2, 3) et le segment de liaison ont la même épaisseur de plaque (Pd1, Pd2).

11. Plaque d'ostéosynthèse selon l'une des revendications précédentes, **caractérisée en ce que** les segments de plaque de fixation (2, 3) ont une étendue longitudinale (l2) et une étendue transversale (b2) perpendiculaire à celle-ci, le segment de liaison étant disposé sur le bord et au milieu par rapport à l'étendue longitudinale (l2).

12. Plaque d'ostéosynthèse selon l'une des revendications précédentes, **caractérisée en ce qu'**une interface (12) entre chaque segment de plaque de fixation (2 ou 3) et le segment de liaison est conformée comme une pliure, de préférence avec un rayon de courbure qui est au moins supérieur à l'épaisseur de plaque moyenne (Pd2) du segment de liaison, de préférence au moins deux fois plus grand.

13. Plaque d'ostéosynthèse selon l'une des revendications précédentes, **caractérisée en ce que** le segment de liaison est bombé et convexe en direction des points de fixation (5) des segments de plaque de fixation (2, 3).
